# EUROPÄISCHE PATENTSCHRIFT

(11) **EP 3 802 204 B1**
(45) Veröffentlichungstag und Bekanntmachung des Hinweises auf die Patenterteilung: **14.12.2022**
(21) Anmeldenummer: 19720517.2
(22) Anmeldetag: 23.04.2019
(51) Int. Cl.: A61B 5/00, B60N 2/02, A61B 5/107

(54) **COMPUTERPROGRAMMPRODUKT ZUM ELEKTRISCHEN EINSTELLEN EINES FAHRZEUGSITZES, EINSTELLSYSTEM UND FAHRZEUG**
COMPUTER PROGRAM FOR THE ELECTRICAL CONFIGURATION OF VEHICLE SEAT, CONFIGURATION SYSTEM AND VEHICLE
PROGRAMME INFORMATIQUE POUR LA CONFIGURAITON ELECTRIQUE D'UNE SIEGE DE VEHICULE, SYSTEME DE CONFIGURATION ET VEHICULE

(30) Priorität: 05.06.2018 DE 102018208837
(43) Veröffentlichungstag der Anmeldung: 14.04.2021
(73) Patentinhaber: VOLKSWAGEN AKTIENGESELLSCHAFT, 38440 Wolfsburg (DE)
(72) Erfinder: BAUR, Elmar, 38489 Jübar (DE); HERDT, Alexej, 38518 Gifhorn (DE); KRUPPA, Ralf, 09224 Chemnitz (DE)
(86) Internationale Anmeldenummer: PCT/EP2019/060391
(87) Internationale Veröffentlichungsnummer: WO 2019/233672

(56) Entgegenhaltungen:
- EP-A1- 2 674 914
- US-A1- 2010 245 555
- US-A1- 2012 086 249
- US-B1- 9 707 912

## Beschreibung

Die vorliegende Erfindung betrifft ein Produkt zum elektrischen Einstellen eines Fahrzeugsitzes. Außerdem betrifft die Erfindung ein Einstellsystem mit einem darauf installierten Produkt zum elektrischen Einstellen eines Fahrzeugsitzes sowie ein Fahrzeug mit einem solchen Einstellsystem.

Im Stand der Technik sind Einstellsysteme zum elektrischen Einstellen von einem oder mehreren Fahrzeugsitzen bekannt. Mittels solcher Einstellsysteme können Fahrzeugsitze durch Knopfdruck und/oder zumindest teilweise automatisierter Prozessabläufe eingestellt werden.

Aus der deutschen Patentanmeldung DE 10 2012 208 644 A1 gehen beispielsweise eine Vorrichtung und ein Verfahren zum Anpassen einer Sitzposition für einen Fahrzeugsitz hervor. Die dargestellte Vorrichtung ermöglicht eine automatische oder automatisierte Anpassung der Sitzposition des betreffenden Fahrzeugsitzes, wobei die Vorrichtung zumindest einen Fahrzeugsitz, zumindest eine erste Sensoreinheit zur Bestimmung von physischen Parametern eines außerhalb des Fahrzeugs befindlichen potentiellen Fahrzeuginsassens und/oder zumindest ein Eingabemittel und/oder zumindest eine zweite Sensoreinheit zur Bestimmung der Sitzposition des Fahrzeugsitzes und zumindest eine Steuereinheit umfasst. Weiterhin wird ein Verfahren zur automatischen oder automatisierten Anpassung einer Sitzposition für einen Fahrzeugsitz mittels der Vorrichtung beschrieben.

Basis für eine gemäß der DE 10 2012 208 644 A1 beschriebene, automatische oder automatisierte Sitzeinstellung ist häufig eine kamerabasierte Erkennung von Gelenkpunkten einer Person bzw. des potentiellen Fahrzeuginsassen. Besonders wichtig ist hierbei die Erkennung des sogenannten H-Punktes (Hüftpunkt). Dies gestaltet sich bei bestimmten Kleidungsstücken jedoch schwierig. Trägt die zu erkennende Person beispielsweise einen Mantel, ein Kleid, einen hochgezogenen Gürtel und eine entsprechend getragene Hose, versagen bisher bekannte Systeme. Die Position des H-Punktes ist jedoch maßgeblich für die Sitzeinstellung.

Aus der US 2012/086249 A1 geht ein System zum elektrischen Einstellen eines Fahrzeugsitzes anhand von Körpermaßen eines Fahrzeuginsassen hervor.

Aufgabe der vorliegenden Erfindung ist es, der voranstehend beschriebenen Problematik bei der Sitzeinstellung in einem Fahrzeug zumindest teilweise Rechnung zu tragen. Insbesondere ist es Aufgabe der vorliegenden Erfindung, ein Produkt, ein Einstellsystem und ein Fahrzeug zur Erzielung einer verbesserten elektrischen Einstellung eines Fahrzeugsitzes zu schaffen.

Die voranstehende Aufgabe wird durch die Patentansprüche gelöst. Insbesondere wird die voranstehende Aufgabe durch das Produkt gemäß Anspruch 1, durch das Einstellsystem gemäß Anspruch 4 sowie durch das Fahrzeug gemäß Anspruch 8 gelöst. Weitere Vorteile der Erfindung ergeben sich aus den Unteransprüchen, der Beschreibung und den Figuren. Dabei gelten Merkmale, die im Zusammenhang mit dem Produkt beschrieben sind, selbstverständlich auch im Zusammenhang mit dem erfindungsgemäßen Einstellsystem mit einem darauf installierten Produkt, dem erfindungsgemäßen Fahrzeug mit dem Einstellsystem und jeweils umgekehrt, sodass bezüglich der Offenbarung zu den einzelnen Erfindungsaspekten stets wechselseitig Bezug genommen wird bzw. werden kann.

Gemäß einem ersten Aspekt der vorliegenden Erfindung wird ein Produkt zum elektrischen Einstellen eines Fahrzeugsitzes und/oder von Peripheriebauteilen wie Außenspiegel, Lenkrad, Sicherheitsgurt sowie Bedienelemente am Fahrzeugsitz, an Konsolen und/oder am Armaturenbrett bereitgestellt. Das Produkt weist ein Anweisungsmittel zum Erstellen einer Anweisung für eine Person zur Einnahme einer vordefinierten Haltung, bei welcher die Person steht und sich wenigstens eine Hand der Person sichtbar auf der Hüfte der Person befindet, auf. Ferner weist das Produkt eine Ermittlungseinheit zum Ermitteln eines H-Punktes der Person anhand der eingenommenen vordefinierten Haltung der Person auf. Darüber hinaus weist das Produkt ein Rechenmittel zum Berechnen einer Sitzeinstellungsinformation basierend auf dem ermittelten H-Punkt sowie ein Signalausgabemittel zum Ausgeben eines Einstellsignals zum elektrischen Einstellen des Fahrzeugsitzes basierend auf der berechneten Sitzeinstellungsinformation auf. Die Ermittlungseinheit ist hierbei zum Ermitteln des H-Punktes anhand einer erkannten Position des Handgelenks der Person und eines zu dieser Position addierten statistischen Wertes konfiguriert.

Die vorgeschlagene Haltung, bei welcher die Person ihre Hand auf ihrer Hüfte bzw. auf ihrem Beckenkamm auflegt, wurde bei umfangreichen Versuchen im Rahmen der vorliegenden Erfindung als besonders vorteilhaft erkannt. Es hat sich gezeigt, dass bei einer Person mit dieser Haltung der H-Punkt besonders genau erkannt werden kann. Durch die sichtbare Positionierung des Handgelenks auf der Hüfte kann verhindert werden, dass Kleidungsstücke, wie lange Oberteile, Mäntel, Kleider oder ein hochgezogener Gürtel, den für die Berechnung der korrekten Sitzposition entscheidenden Hüftbereich überdecken und dadurch kein Referenzpunkt zur Ermittlung des H-Punktes ermittelt werden kann. Die wenigstens eine Hand der Person befindet sich vorzugsweise dann sichtbar auf der Hüfte der Person, wenn die Hand auf einem Foto, das von der Vorderseite der Person geschossen wird, möglichst vollständig erkennbar ist.

Zur Ermittlung des H-Punktes ist die Ermittlungseinheit zur Analyse der Person, welche die vordefinierte Haltung angenommen hat, konfiguriert. Die Analyse kann beispielsweise anhand eines Fotos der Person, welche die vordefinierte Haltung angenommen hat, durchgeführt werden. Die Analyse kann auch in Echtzeit durchgeführt werden, d.h., anhand von Bildmaterial, das durch eine mit der Ermittlungseinheit in Signalverbindung stehenden Kamera aufgenommen wird.

Im Rahmen von umfangreichen Versuchen wurde erkannt, dass von einer Position des Handgelenks auf der Hüfte der Person mit statistischen Mitteln relativ zuverlässig und genau auf die Position des gesuchten H-Punktes geschlossen werden kann. Unter dem statistischen Wert kann eine Länge verstanden werden, die abhängig von der erkannten Person und entsprechenden zusätzlichen Referenzwerten unterschiedlich ausfallen kann. Bei kleinen Personen, großen Personen, Männern und Frauen können beispielsweise unterschiedliche statistische Werte bzw. Längen verwendet werden. Der statistische Wert kann sich beispielsweise in einem Bereich zwischen 1 cm und 10 cm befinden. Es ist aber auch denkbar, dass der Bereich größer als 10 cm ist, beispielsweise zwischen 10 cm und 20 cm. D.h., für die Ermittlung des H-Punktes kann zur erkannten Position des Handgelenks noch eine abhängig von der erkannten Person vordefinierte Länge addiert werden.

Die Anweisung zur Einnahme der vordefinierten Haltung wird vorzugsweise dahingehend erstellt, dass ein Daumen der wenigstens einen Hand der Person nicht versteckt in Richtung des Rückens der Person, sondern in Richtung der Seite, des Beckenkamms, der Füße und/oder des Bauchs der Person zeigt. Insbesondere soll der Daumen der wenigstens einen Hand der Person derart positioniert werden, dass er auf einem Foto, das von der Vorderseite der Person geschossen wird, erkannt werden kann.

Von besonderem Vorteil ist es, wenn das Anweisungsmittel zum Erstellen einer Anweisung für die Person zur Einnahme einer vordefinierten Haltung konfiguriert ist, bei welcher die Person steht und sich beide Hände der Person sichtbar auf der Hüfte der Person befinden. Wenn sich beide Hände auf der Hüfte der Person befinden, kann die Wahrscheinlichkeit, dass trotzdem noch ein Kleidungsstück einen Bereich der Person zur Ermittlung des H-Punktes verdeckt, auf ein Minimum reduziert werden. Entsprechend zuverlässig und genau kann anhand dieser Haltung der H-Punkt ermittelt werden. Ebenso genau kann anschließend der Fahrzeugsitz eingestellt werden.

Die Anweisungsvorrichtung kann ferner konfiguriert sein, einen Hinweis für die Person dahingehend zu erstellen, dass die Hand nicht durch den Körper der Person, ein Kleidungsstück oder einen anderen Gegenstand verdeckt sein darf oder dass wenigstens eine Hand der Person auf einem Foto, das von der Vorderseite der Person geschossen wird, frei sichtbar sein muss. Dies kann in Form einer akustischen und/oder grafischen Anweisung erstellt werden. So ist es denkbar, dass das Anweisungsmittel zum Erstellen einer entsprechenden Sprachanweisung konfiguriert ist. Zusätzlich oder alternativ kann das Anweisungsmittel zum Erstellen einer bildlichen Darstellung konfiguriert sein, auf welcher der Person ein Hinweis zum Zeigen der Hand angezeigt wird.

Das Produkt ist zum elektrischen, vorzugsweise automatischen und/oder automatisierten Einstellen des Fahrzeugsitzes konfiguriert. D.h., sobald das Produkt aktiviert wurde, braucht die Person grundsätzlich nur noch den erstellten und anschließend angezeigten Anweisungen folgen, um die gewünschten Einstellsignale zum Einstellen des Fahrzeugsitzes in die gewünschte Sitzposition zu erzeugen.

Die Ermittlungseinheit kann nicht nur zur Ermittlung des H-Punktes, sondern auch zur Ermittlung anderer Referenzpunkte der Person konfiguriert sein. So kann die Ermittlungseinheit zur Ermittlung von Referenzpunkten, wie Kopf, Schultern, Ellenbogen, Knie und/oder Füßen, konfiguriert sein. Anhand dieser Referenzpunkte kann ggf. nicht nur auf den H-Punkt geschlossen werden. Vielmehr können auch diese Referenzpunkte, wie vorstehend beschrieben, zum Berechnen der Sitzeinstellungsinformation und folglich zur Erstellung des Einstellsignals verwendet werden. Die Ermittlungseinheit ist demnach insbesondere zur indirekten Erkennung des H-Punktes ausgestaltet.

Das erfindungsgemäße Produkt ist vorzugsweise zum elektrischen Einstellen von wenigstens einem Fahrzeugsitz konfiguriert. So kann das Produkt beispielsweise nur zum elektrischen Einstellen des Fahrersitzes oder zum elektrischen Einstellen aller Fahrzeugsitze des Fahrzeugs konfiguriert sein.

Das Anweisungsmittel ist zum Einstellen einer Anweisung für wenigstens eine Person konfiguriert. D.h., wenn das Produkt zum elektrischen Einstellen aller Fahrzeugsitze des Fahrzeugs konfiguriert ist, kann das Anweisungsmittel auch zum Einstellen einer Anweisung für mehrere Personen zur jeweiligen Einnahme einer vordefinierten Haltung, bei welcher jede Person steht und sich jeweils wenigstens eine Hand der jeweiligen Person sichtbar auf der Hüfte dieser Person befindet, konfiguriert sein. Die Anweisung kann noch Hinweise zur Aufstellung der Personen enthalten. So kann eine Anweisung zu einer Aufstellungsreihenfolge und/oder Aufstellungsanordnung der Personen erstellt werden, anhand welcher ein Zuordnungsmittel die zu den jeweiligen Personen erfassten H-Punkte verschiedenen Sitzen im Fahrzeug zuordnet. So kann ferner zwischen einem Fahrersitz, einem Beifahrersitz und einer Rücksitzbank unterschieden werden. Wird nun erkannt, dass sich eine große Person auf den Fahrersitz setzten möchte, eine kleine Person auf den Beifahrersitz und große Personen auf die Rücksitzbank, können die jeweiligen Sitzeinstellungsinformationen entsprechend berechnet und über das Signalausgabemittel zum Einstellen der Fahrzeugsitze ausgegeben werden.

Unter der Sitzeinstellungsinformation ist wenigstens eine Sitzeinstellungsinformation zu verstehen. Insbesondere können mehrere Sitzeinstellungsinformationen dahingehend berechnet werden, dass verschiedene Sitzabschnitte unterschiedlich eingestellt werden. So kann das Signalausgabemittel zum Ausgeben eines Einstellsignals zum elektrischen Einstellen des Fahrzeugsitzes bzw. verschiedener Komponenten des Fahrzeugsitzes, wie Sitzbereich, Rückenlehne, Lordosestütze, Armlehne, Kopfstütze und/oder die allgemeine Relativposition des Fahrzeugsitzes im Fahrzeug, basierend auf den berechneten Sitzeinstellungsinformationen konfiguriert sein.

Das Rechenmittel ist zum Berechnen der Sitzeinstellungsinformation anhand eines vordefinierten Algorithmus und/oder entsprechend geeigneter Rechenfunktionen konfiguriert.

Das Produkt kann als computerlesbarer Anweisungscode in jeder geeigneten Programmiersprache, wie beispielsweise in JAVA, C++ und/oder C#, insbesondere in einer Smart Device wie einem Smartphone, implementiert sein Das Produkt kann auf einem computerlesbaren Speichermedium, wie einer Datendisk, einem Wechsellaufwerk, einem flüchtigen oder nichtflüchtigen Speicher, einem eingebauten Speicher/Prozessor, einem Datenbank Backend, oder einer Smart Device abgespeichert sein. Der Anweisungscode kann einen Computer oder andere programmierbare Geräte, wie ein Steuergerät, derart programmieren, dass die gewünschten Funktionen ausgeführt werden. Ferner kann das Produkt in einem Netzwerk, wie beispielsweise dem Internet, bereitgestellt werden bzw. sein, von dem es bei Bedarf von einem Nutzer heruntergeladen werden kann. Das Produkt kann sowohl mittels eines Computerprogramms, d.h. einer Software, als auch mittels einer oder mehrerer spezieller elektronischer Schaltungen, d.h. in Hardware, oder in beliebig hybrider Form, d.h. mittels Software-Komponenten und Hardware-Komponenten, realisiert werden bzw. sein.

Bei einem erfindungsgemäßen Produkt ist es außerdem möglich, dass das Anweisungsmittel zum Erstellen einer digitalen Maske der vordefinierten Haltung und zum Erstellen der Anweisung für die Person zur Einnahme der vordefinierten Haltung innerhalb der digitalen Maske konfiguriert ist. Unter der digitalen Maske kann eine Silhouette oder Schablone verstanden werden, welche die Umrisse einer Person, welche die vordefinierte Haltung einnimmt, darstellt. Die Person kann sich zur Ermittlung des H-Punktes derart positionieren bzw. hinstellen, dass sie sich möglichst genau in der erstellten und entsprechend angezeigten digitalen Maske befindet. Die beschriebene Vorgehensweise ist mit dem Erstellen eines biometrischen Passbildes vergleichbar, zur Erstellung von welchem eine Person ihren Kopf ebenfalls in eine digitale Maske in Form eines Kopfes einer Person bewegen bzw. dort korrekt positionieren soll. Mit Hilfe der digitalen Maske fällt es der Person erheblich leichter, die gewünschte bzw. geforderte Haltung einzunehmen. Ausgehend von einer entsprechend präzisen Haltung der Person können selbstverständlich auch der H-Punkt entsprechend präzise bestimmt und die erforderliche Sitzeinstellungsinformation genau berechnet werden. Zur Ermittlung von verschiedenen H-Punkten von verschiedenen Personen kann das Anweisungsmittel ferner konfiguriert sein, eine digitale Maske für mehrere Personen sowie eine Anweisung für die Personen zur jeweiligen Einnahme der vordefinierten Haltung innerhalb der digitalen Maske zu erstellen. Mit Hilfe dieses Vorgehens bzw. dieser Konfiguration des Produkts können sämtliche Sitze oder Sitzreihen des Fahrzeugs auf schnelle und einfache Weise in die gewünschte Position gestellt werden. Die digitale Maske ist auf verschiedene Körpergrößen und Proportionen individuell anpassbar. So kann das Anweisungsmittel zum Erstellen einer individuellen digitalen Maske anhand einer erkannten Größe und/oder Proportion einer Person konfiguriert sein. Die digitale Maske kann hierfür entsprechend vergrößert oder verkleinert werden.

Darüber hinaus ist es bei einem Produkt gemäß der vorliegenden Erfindung möglich, dass das Produkt in Form einer Applikation zum Ausführen auf einer Smart-Device implementiert ist. D.h., das Produkt ist konfiguriert, sich grundsätzlich mit jeder aktuellen Smart Device ausführen zu lassen. Die erstellte Anweisung kann in diesem Fall beispielsweise auf einem Display der Smart Device angezeigt werden. Die Person, die üblicherweise ohnehin eine geeignete Smart Device besitzt, kann das erfindungsgemäße Produkt dadurch besonders einfach und kostengünstig zum elektrischen Einstellen des Fahrzeugsitzes verwenden. Unter einer Smart Device können ein Smartphone, ein Tablet-Computer und/oder eine Smart-Watch verstanden werden.

Gemäß einem weiteren Aspekt der vorliegenden Erfindung wird ein Einstellsystem mit einem darauf installierten, wie vorstehend im Detail erläuterten Produkt zum elektrischen Einstellen eines Fahrzeugsitzes zur Verfügung gestellt. Das Einstellsystem weist eine Signalübertragungseinheit zum Übertragen des Einstellsignals zum Einstellen des Fahrzeugsitzes zu dem Fahrzeug auf. In dem Einstellsystem sind als Bestandteile des Produkts das Anweisungsmittel zum Erstellen einer Anweisung für eine Person zur Einnahme einer vordefinierten Haltung, die Ermittlungseinheit zum Ermitteln eines H-Punktes der Person anhand der eingenommenen vordefinierten Haltung der Person, das Rechenmittel zum Berechnen einer Sitzeinstellungsinformation basierend auf dem ermittelten H-Punkt, und das Signalausgabemittel zum Ausgeben eines Einstellsignals zum elektrischen Einstellen des Fahrzeugsitzes basierend auf der berechneten Sitzeinstellungsinformation, ausgestaltet. Das Einstellsystem kann ferner eine Eingabemaske für die manuelle Eingabe von verschiedenen personenbezogenen Daten, wie beispielsweise die Körpergröße, Armlängen, oder Beinlängen, aufweisen. Damit bringt das erfindungsgemäße Einstellsystem die gleichen Vorteile mit sich, wie sie ausführlich mit Bezug auf das erfindungsgemäße Produkt beschrieben worden sind. Unter dem Einstellsystem kann eine wie vorstehend beschriebene Smart Device oder ein in ein Fahrzeug integriertes Einstellsystem verstanden werden. Die Signalübertragungseinheit kann in Form eines Funksenders oder in Form eines kabelgebundenen Signalsenders ausgestaltet sein. Ein erfindungsgemäßer Funksender kann ein WLAN-Modul, ein Bluetooth-Modul und/oder ein RFID-Modul zum Übertragen des Einstellsignals zum Einstellen des Fahrzeugsitzes zu dem Fahrzeug aufweisen.

Bei einer erfindungsgemäßen Ausgestaltungsvariante ist es möglich, dass eine Anzeigevorrichtung zum Anzeigen der durch das Anweisungsmittel erstellten Anweisung zur Einnahme der vordefinierten Haltung und/oder der digitalen Maske bereitgestellt ist. Die Anzeigevorrichtung ist vorzugsweise in Form eines Displays bzw. Bildschirms bereitgestellt. Für den Fall, dass das Einstellsystem eine Smart Device aufweist oder als solche ausgestaltet ist, kann die Anzeigevorrichtung dem Display der Smart Device entsprechen. Gleichwohl kann die Anzeigevorrichtung auch dem Fahrzeug zugeordnet sein. In diesem Fall kann die Anzeigevorrichtung einem Display entsprechen, das in dem Fahrzeug angeordnet ist, insbesondere dem Display des Navigationssystems bzw. einem Display im Armaturenbrett des Fahrzeugs.

Weiterhin ist es möglich, dass bei einem Einstellsystem gemäß der vorliegenden Erfindung eine Kamera zum Fotografieren der Person bereitgestellt ist, wobei die Anweisungseinheit konfiguriert ist, eine Positionsempfehlung für die Kamera zum Fotografieren der Person zu erstellen und diese mit einer Abstandsangabe und/oder einer Winkelangabe der Kamera zur Person und/oder zum Boden auf einer Anzeigevorrichtung des Einstellsystems anzuzeigen. Wenn das Einstellsystem in Form einer Smart Device ausgestaltet ist, entspricht die Kamera der Smart Device Kamera. Die Anzeigevorrichtung entspricht in diesem Fall dem Display der Smart Device. Bei umfangreichen Versuchen im Rahmen der vorliegenden Erfindung hat sich herausgestellt, dass mit einer bestimmten Ausrichtung und Beabstandung der Kamera zur Person hinsichtlich der Ermittlung des H-Punktes besonders aussagekräftige Fotos der Person geschossen werden können. Würde die Kamera beispielsweise zu nahe an der Person aufgestellt werden, wären entweder Teile der Person auf dem Foto abgeschnitten und könnten für die Auswertung des Fotos nicht mehr herangezogen werden, oder es müsste ein geeignetes Weitwinkelobjektiv verwendet werden, welchen dann jedoch zu Verzeichnungen im Randbereich des Fotos führen würde, welche zu Fehlinterpretationen hinsichtlich des H-Punktes führen könnten. Selbiges trifft zu, wenn die Kamera beim Fotografieren seitlich, zu hoch oder zu niedrig zur Person positioniert wird. Auch in diesem Fall können Bildverzeichnungen auf dem Foto auftreten, die ein genaues Ermitteln des H-Punktes erschweren oder unmöglich machen. Bei einem zu großen Abstand zwischen der Person und der Kamera kann die relative Pixeldichte der Person auf dem Foto zu gering ausfallen, um noch aussagekräftige Referenzpunkte zur Ermittlung des H-Punktes aus dem Foto ableiten zu können. Als bevorzugte Position der Kamera hat sich eine Stelle mittig oder im Wesentlichen mittig vor der Person, also auf einer Höhe von ca. 0,5m bis 1,5m vom Boden und/oder von einem Fluchtpunt ca. in der Mitte des Körpers der Person mit einem Abstand in einem Bereich zwischen 1m und 3m, insbesondere zwischen 1,5m und 2,5m, besonders bevorzugt ca. 2m, herausgestellt. Mit einer geeigneten Optik, beispielsweise einem Teleobjektiv, und/oder einem geeigneten Digitalzoom kann ein noch größerer Abstand von beispielsweise 3m bis 5m von Vorteil sein. Verzeichnungen im Randbereich der Bildaufnahme können hierbei nochmals reduziert werden. Entsprechend kann auch die Positionsempfehlung erstellt werden. D.h., die Anweisungseinheit kann zum Erstellen einer entsprechenden Positionsempfehlung konfiguriert sein. Für den Fall, dass das Foto trotz korrekter Positionierung der Person zur Kamera störende Verzeichnungen aufweist, welche durch die Optik der Kamera bedingt sein können, kann das Produkt ein Verzeichnungskorrekturmittel zur automatischen Verzeichnungskorrektur aufweisen. Die Kamera kann bei einer bevorzugten Weiterbildung als 3D-Kamera, insbesondere TOF-Kamera, ausgestaltet sein. Das Einstellsystem kann ferner eine Überwachungseinheit zur Überwachung des vorgegebenen Winkels und/oder Abstands der Kamera zur Person und/oder zum Boden aufweisen, wobei die Überwachungseinheit konfiguriert ist, ein Hinweissignal zu erstellen und/oder auszugeben, wenn eine vordefinierte Abweichung vom vorgegebenen Winkel und/oder Abstand erkannt wird.

Ein erfindungsgemäßes Einstellsystem kann ferner dahingehend bereitgestellt sein, dass das Anweisungsmittel zum Erstellen und Anzeigen einer Bestätigung einer korrekt eingenommenen Haltung und/oder zum Erstellen und Anzeigen eines Hinweises auf eine nicht korrekt eingenommene Haltung auf der Anzeigevorrichtung konfiguriert ist. So kann der Person akustisch, visuell und/oder haptisch mitgeteilt werden, dass die eingenommene Position relativ zur Kamera korrekt oder eben nicht korrekt ist. Fehler bei der Aufzeichnung des Fotos können damit verhindert werden. Das Einstellsystem kann ferner eine Selbstauslösevorrichtung aufweisen, welche eine vordefinierte Einstellung der Kamera, beispielsweise des Zooms, sowie das Fotografieren der Person bei Erkennen einer vordefinierten Person automatisch vornimmt. Dies ist beispielsweise durch eine Gesichtserkennungseinheit realisierbar.

Gemäß einem weiteren Aspekt der vorliegenden Erfindung wird ein Fahrzeug zur Verfügung gestellt, das zum elektrischen Einstellen eines Fahrzeugsitzes des Fahrzeugs mit einem wie vorstehend im Detail erläuterten Einstellsystem konfiguriert ist. Das Fahrzeug weist eine Signalempfangseinheit zum Empfangen des Einstellsignals sowie eine Sitzeinstelleinheit, die zum elektrischen Einstellen des Fahrzeugsitzes basierend auf dem Einstellsignal mit der Signalempfangseinheit in Signalverbindung steht, auf. Damit bringt auch das erfindungsgemäße Fahrzeug die vorstehend beschriebenen Vorteile mit sich. Das Fahrzeug ist vorzugsweise in Form eines Straßenfahrzeugs, wie eines PKWs oder LKWs, ausgestaltet. Das Fahrzeug kann jedoch auch ein Luftfahrzeug, Wasserfahrzeug, Schienenfahrzeug oder ein Roboter sein. Die Signalempfangseinheit kann in Form einer drahtlosen oder drahtgebundenen Signalempfangseinheit bereitgestellt sein. Vorzugsweise ist die Signalempfangseinheit als drahtlose Signalempfangseinheit, beispielsweise mit einem WLAN-Empfänger, einem Bluetooth-Empfänger und/oder einem RFID-Empfänger bzw. jeweils einem entsprechenden Funk-Modul ausgestaltet.

Bei einem Fahrzeug gemäß der vorliegenden Erfindung ist es zudem möglich, dass die Kamera im Außenbereich des Fahrzeugs integriert ist. Auf eine externe Vorrichtung wie die vorstehend beschriebene Smart Device könnte in diesem Fall verzichtet werden. Wenn sich Personen, die das Einstellsystem bereits häufiger genutzt haben und wissen, wie sie sich vor der Kamera zu positionieren haben, vor dem Fahrzeug stehen, wollen diese Personen ggf. nicht auf ihre Smart Device angewiesen sein. So ist es denkbar, dass das Fahrzeug beispielsweise im Türbereich, insbesondere im Seitenspiegel, in oder an der Fahrertüre, vorzugsweise in einer Höhe in einem Bereich zwischen 0,5m und 1,5m vom Boden, die Kamera zum Fotografieren der Person oder einer Personengruppe installiert hat. Die Kamera könnte mit einem Personenerkennungsmittel ausgestattet sein, sodass die Person, die vor dem Fahrzeug die vordefinierte Haltung einnimmt, automatisch fotografiert wird und basierend auf den Referenzpunkten, die auf dem Foto erkannt werden, der Fahrzeugsitz automatisch eingestellt wird. Hilfsweise kann der Auslöser zum Schießen des Fotos auch durch eine Applikation auf einer Smart Device betätigt werden. D.h., das Produkt und damit auch das Einstellsystem könnten ein entsprechendes Auslösemittel zum Schießen des Fotos aufweisen.

Weitere, die Erfindung verbessernde Maßnahmen ergeben sich aus der nachfolgenden Beschreibung zu verschiedenen Ausführungsbeispielen der Erfindung, welche in den Figuren schematisch dargestellt sind.

Es zeigen jeweils schematisch:
- Figur 1: ein Einstellsystem gemäß einer Ausführungsform der vorliegenden Erfindung,
- Figur 2: ein erfindungsgemäßes Fahrzeug, das zum elektrischen Einstellen eines Fahrzeugsitzes mit einem wie in Fig. 1 dargestellten Einstellsystem konfiguriert ist,
- Figuren 3 bis 5: ein auf einer Anzeigevorrichtung ausgeführtes Produkt gemäß einer Ausführungsform der vorliegenden Erfindung, und
- Figur 6: eine Darstellung zum Erläutern einer erfindungsgemäßen Ermittlung eines H-Punktes einer Person.

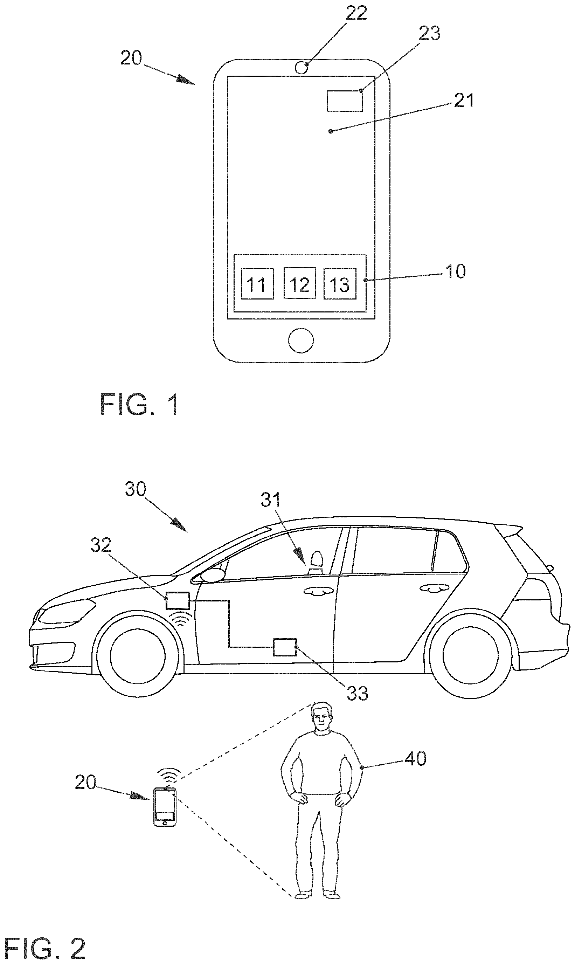

Elemente mit gleicher Funktion und Wirkungsweise sind in den Figuren 1 bis 6 jeweils mit denselben Bezugszeichen versehen.

Mit Bezug auf Fig. 1 und Fig. 2 wird ein Einstellsystem 20 in Form eines Smartphones mit einem darauf installierten Produkt 10 zum elektrischen Einstellen eines Fahrzeugsitzes 31 eines Fahrzeugs 30 erläutert. Das Produkt 10 weist ein Anweisungsmittel 11 zum Erstellen einer Anweisung für eine Person 40 zur Einnahme einer vordefinierten Haltung auf, bei welcher die Person 40 steht und sich beide Hände der Person 40 sichtbar auf der Hüfte der Person 40 befinden. Das Produkt 10 weist ferner eine Ermittlungseinheit 12 zum Ermitteln eines H-Punktes H der Person 40 anhand der eingenommenen vordefinierten Haltung der Person 40 bzw. eines entsprechenden Fotos, das von der Person 40 in dieser Haltung geschossen wurde, auf. Außerdem weist das Produkt 10 ein Rechenmittel 12 zum Berechnen einer Sitzeinstellungsinformation basierend auf dem ermittelten H-Punkt H auf. Darüber hinaus weist das Produkt 10 ein Signalausgabemittel 13 zum Ausgeben eines Einstellsignals zum elektrischen Einstellen des Fahrzeugsitzes 31 basierend auf der berechneten Sitzeinstellungsinformation auf. Das dargestellte Produkt 10 ist vorliegend in Form einer Applikation zum Ausführen auf dem als Smartphone ausgestalteten Einstellsystem 20 implementiert.

Das Fahrzeug 30 weist eine Signalempfangseinheit 32 zum Empfangen des Einstellsignals sowie eine Sitzeinstelleinheit 33, die zum elektrischen Einstellen des Fahrzeugsitzes 31 basierend auf dem Einstellsignal mit der Signalempfangseinheit 32 in Signalverbindung steht, auf. Die Signalempfangseinheit 32 ist für eine Signalübertragung zwischen der Signalempfangseinheit 32 und der Sitzeinstelleinheit 33 mit einem Kabel mit der Sitzeinstelleinheit 33 verbunden. Zur Übertragung der Sitzeinstellungsinformation von der Signalübertragungseinheit 23 zu der Signalempfangseinheit 32 weisen sowohl die Signalübertragungseinheit 23 als auch die Signalempfangseinheit 32 ein geeignetes WLAN-Modul auf.

Das dargestellte Einstellsystem 20 weist eine Kamera 22 zum Fotografieren der Person auf. Außerdem weist das Einstellsystem 20 eine Anzeigevorrichtung 21 in Form eines Displays zum Anzeigen der durch das Anweisungsmittel 11 erstellten Anweisung zur Einnahme der vordefinierten Haltung auf.

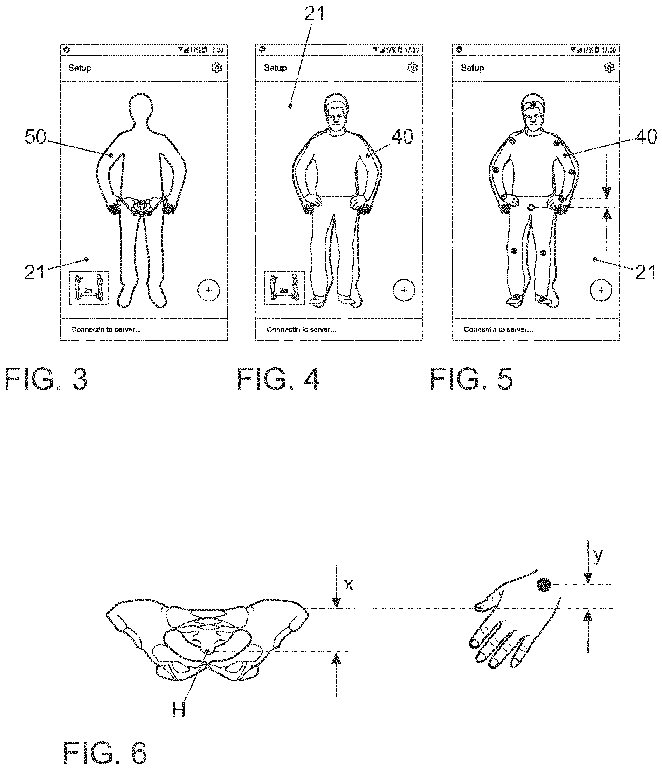

Mit Bezug auf die Figuren 3 bis 6 wird anschließend eine Anwendungsvariante des Einstellsystems 20 erläutert. Die Kamera 22 des Einstellsystems 20 ist hierbei zum Fotografieren der Person 40 bereitgestellt, wobei die Anweisungseinheit 11 konfiguriert ist, eine Positionsempfehlung für die Kamera 22 zum Fotografieren der Person 40 zu erstellen und diese mit einer Abstandsangabe zwischen der Kamera 22 und der zu fotografierenden Person auf der Anzeigevorrichtung 21 des Einstellsystems 20 anzuzeigen. Ferner ist das Anweisungsmittel 11 zum Erstellen einer digitalen Maske 50 der vordefinierten Haltung und zum Erstellen der Anweisung für die Person 40 zur Einnahme der vordefinierten Haltung innerhalb der digitalen Maske konfiguriert.

Die Person 40, die sich nun in die angewiesene Position bringen soll, kann das Einstellsystem 20 bzw. das Smartphone mit der Kamera 22 nun beispielsweise einer weiteren Person geben, die anschließen das benötigte Foto der Person 40 innerhalb der digitalen Maske 50 schießt. Ein derart erstelltes Foto ist in Fig. 4 dargestellt. Hilfsweise könnte dieses Foto auch durch einen Selbstauslöser des Smartphones oder eine im Fahrzeug 30 integrierte Kamera, die durch das Smartphone betätigbar ist, erstellt werden.

Sobald das Foto der Person innerhalb der digitalen Maske geschossen wurde, kann die Ermittlungseinheit 12 den H-Punkt H anhand einer erkannten Position des Handgelenks der Person 40 und eines zu dieser Position addierten statistischen Wertes ermittelt werden. Dies ist insbesondere in Fig. 5 und vergrößert in Fig. 6 dargestellt. Neben dem H-Punkt bzw. dem dafür ermittelten Referenzpunkt am Handgelenk der Person können, wie in Fig. 5 dargestellt, noch weitere Referenzpunkte ermittelt werden, welche ebenfalls als Grundlage für die gewünschte Sitzeinstellung und/oder für die Ermittlung des H-Punktes H verwendet werden können.

Die Erfindung lässt neben den dargestellten Ausführungsformen weitere Gestaltungsgrundsätze zu. D.h., die Erfindung soll nicht auf die mit Bezug auf die Figuren erläuterten Ausführungsbeispiele beschränkt betrachtet werden.

So braucht beispielsweise nicht die Kamera 22 des Smartphones für das benötigte Foto verwendet werden. Alternativ kann auch eine Kamera verwendet werden, die im Außenbereich des Fahrzeugs 30, beispielsweise im Türbereich des Fahrzeugs 30, installiert ist. Das Anweisungsmittel 11 kann ferner zum Erstellen und Anzeigen einer Bestätigung einer korrekt eingenommenen Haltung und/oder zum Erstellen und Anzeigen eines Hinweises auf eine nicht korrekt eingenommene Haltung auf der Anzeigevorrichtung 21 konfiguriert sein. Dies kann beispielsweise mit einem grünen Haken für eine korrekte Haltung und einem roten Kreuz für eine nicht korrekt eingenommene Haltung, oder vergleichbaren Mitteln, umgesetzt werden. Außerdem ist es möglich, dass auf der Anzeigevorrichtung 21 nicht nur der gewünschte bzw. geforderte Abstand zwischen Kamera 22 und Person 40, sondern auch noch eine Anweisung zur Ausrichtung der Kamera 22 zur Person auf einer Anzeigevorrichtung 21 des Einstellsystems 20 angezeigt wird bzw. das Produkt 10 zum Erstellen einer entsprechenden Anweisung konfiguriert ist.

### Bezugszeichenliste

- 10: Produkt
- 11: Anweisungsmittel
- 12: Ermittlungseinheit
- 13: Signalausgabemittel
- 20: Einstellsystem
- 21: Anzeigevorrichtung
- 22: Kamera
- 23: Signalübertragungseinheit
- 30: Fahrzeug
- 31: Fahrzeugsitz
- 32: Signalempfangseinheit
- 33: Sitzverstelleinheit
- 40: Person
- 50: digitale Maske

## Patentansprüche

1. Produkt (10) zum elektrischen Einstellen eines Fahrzeugsitzes (31), aufweisend ein Anweisungsmittel (11) zum Erstellen einer Anweisung für eine Person (40) zur Einnahme einer vordefinierten Haltung, bei welcher die Person (40) steht und sich wenigstens eine Hand der Person (40) sichtbar auf der Hüfte der Person (40) befindet, eine Ermittlungseinheit (12) zum Ermitteln eines H-Punktes (H) der Person (40) anhand der eingenommenen vordefinierten Haltung der Person (40), ein Rechenmittel (12) zum Berechnen einer Sitzeinstellungsinformation basierend auf dem ermittelten H-Punkt (H), und ein Signalausgabemittel (13) zum Ausgeben eines Einstellsignals zum elektrischen Einstellen des Fahrzeugsitzes (31) basierend auf der berechneten Sitzeinstellungsinformation,
**dadurch gekennzeichnet,**
**dass** die Ermittlungseinheit (12) zum Ermitteln des H-Punktes (H) anhand einer erkannten Position des Handgelenks der Person (40) und eines zu dieser Position addierten statistischen Wertes konfiguriert ist.

2. Produkt (10) nach Anspruch 1,
**dadurch gekennzeichnet,**
das Anweisungsmittel (11) zum Erstellen einer digitalen Maske (50) der vordefinierten Haltung und zum Erstellen der Anweisung für die Person (40) zur Einnahme der vordefinierten Haltung innerhalb der digitalen Maske konfiguriert ist.

3. Produkt (10) nach einem der voranstehenden Ansprüche,
**dadurch gekennzeichnet,**
**dass** das Produkt (10) in Form einer Applikation zum Ausführen auf einer Smart-Device implementiert ist.

4. Einstellsystem (20) mit einem darauf installierten Produkt (10) nach einem der voranstehenden Ansprüche zum elektrischen Einstellen eines Fahrzeugsitzes (31), aufweisend eine Signalübertragungseinheit (23) zum Übertragen des Einstellsignals zum Einstellen des Fahrzeugsitzes (31) zu dem Fahrzeug (30).

5. Einstellsystem (20) nach Anspruch 4,
**dadurch gekennzeichnet,**
**dass** eine Anzeigevorrichtung (21) zum Anzeigen der durch das Anweisungsmittel (11) erstellten Anweisung zur Einnahme der vordefinierten Haltung und/oder der digitalen Maske (50) bereitgestellt ist.

6. Einstellsystem (20) nach einem der Ansprüche 4 bis 5,
**dadurch gekennzeichnet,**
**dass** eine Kamera (22) zum Fotografieren der Person (40) bereitgestellt ist, wobei die Anweisungseinheit (11) konfiguriert ist, eine Positionsempfehlung für die Kamera (22) zum Fotografieren der Person (40) zu erstellen und diese mit einer Abstandsangabe und/oder einer Winkelangabe der Kamera (22) zur Person und/oder zum Boden auf einer Anzeigevorrichtung (21) des Einstellsystems (20) anzuzeigen.

7. Einstellsystem (20) nach einem der Ansprüche 4 bis 6,
**dadurch gekennzeichnet,**
**dass** das Anweisungsmittel (11) zum Erstellen und Anzeigen einer Bestätigung einer korrekt eingenommenen Haltung und/oder zum Erstellen und Anzeigen eines Hinweises auf eine nicht korrekt eingenommene Haltung auf der Anzeigevorrichtung (21) konfiguriert ist.

8. Fahrzeug (30), das zum elektrischen Einstellen eines Fahrzeugsitzes (31) des Fahrzeugs (30) mit einem Einstellsystem (20) nach einem der Ansprüche 4 bis 7 konfiguriert ist, aufweisend eine Signalempfangseinheit (32) zum Empfangen des Einstellsignals sowie eine Sitzeinstelleinheit (33), die zum elektrischen Einstellen des Fahrzeugsitzes (31) basierend auf dem Einstellsignal mit der Signalempfangseinheit (32) in Signalverbindung steht.

9. Fahrzeug (30) nach Anspruch 8,
**dadurch gekennzeichnet,**
die Kamera (22) im Außenbereich des Fahrzeugs (30) integriert ist.

## Claims

1. Product (10) for electrically configuring a vehicle seat (31), comprising an instruction means (11) for creating an instruction for a person (40) to assume a predefined posture in which the person (40) is standing and at least one hand of the person (40) is visible on the hip of the person (40), a determination unit (12) for determining an H point (H) of the person (40) based on the assumed predefined posture of the person (40), a computing means (12) for calculating seat configuration information based on the determined H-point (H), and a signal output means (13) for outputting an configuration signal for electrically configuring the vehicle seat (31) on the basis of the calculated seat configuration information
**characterized in that**
the determination unit (12) is configured to determine the H point (H) on the basis of a detected position of the wrist of the person (40) and a statistical value added to this position.

2. Product (10) according to Claim 1,
**characterized in that**
the instruction means (11) is configured to create a digital mask (50) of the predefined posture and to create the instruction for the person (40) to assume the predefined posture within the digital mask.

3. Product (10) according to either of the preceding claims,
**characterized in that**
the product (10) is implemented in the form of an application for execution on a smart device.

4. Configuration system (20) with a product (10) installed thereon according to any one of the preceding claims for electrically configuring a vehicle seat (31), comprising a signal transmission unit (23) for transmitting to the vehicle (30) the configuration signal for configuring the vehicle seat (31).

5. Configuration system (20) according to Claim 4,
**characterized in that**
a display device (21) is provided for displaying the instruction created by the instruction means (11) for assuming the predefined posture and/or the digital mask (50).

6. Configuration system (20) according to Claim 4 or Claim 5,
**characterized in that**
a camera (22) for photographing the person (40) is provided, wherein the instruction unit (11) is configured to create a position recommendation for the camera (22) for photographing the person (40) and on a display device (21) of the configuration system (20) to display said position recommendation with a distance indication and/or an angle indication of the camera (22) in relation to the person and/or the ground.

7. Configuration system (20) according to any one of Claims 4 to 6,
**characterized in that**
the instruction means (11) is configured for creating and displaying on the display device (21) a confirmation of a correctly adopted posture and/or for creating and displaying an indication of an incorrectly adopted posture.

8. Vehicle (30) configured to electrically adjust a vehicle seat (31) of the vehicle (30) with a configuration system (20) according to any one of Claims 4 to 7, comprising a signal receiving unit (32) for receiving the configuration signal and a seat configuration unit (33) that is in signal connection with the signal receiving unit (32) for electrically configuring the vehicle seat (31) on the basis of the configuration signal.

9. Vehicle (30) according to Claim 8,
**characterized in that**
the camera (22) is integrated in the exterior region of the vehicle (30).

## Revendications

1. Produit (10) pour le réglage électrique d'un siège de véhicule (31), présentant un moyen de consigne (11) pour créer une consigne pour une personne (40) afin qu'elle prenne une posture prédéfinie, dans laquelle la personne (40) est debout et au moins une main de la personne (40) se trouve de manière visible sur la hanche de la personne (40), une unité de détermination (12) pour déterminer un point H (H) de la personne (40) à l'aide de la posture prédéfinie prise par la personne (40), un moyen de calcul (12) pour calculer une information de réglage de siège sur la base du point H (H) déterminé et un moyen de sortie de signal (13) pour sortir un signal de réglage pour le réglage électrique du siège de véhicule (31) sur la base de l'information de réglage de siège calculée
**caractérisé en ce**
**que** l'unité de détermination (12) est configurée pour déterminer le point H (H) à l'aide d'une position reconnue du poignet de la personne (40) et d'une valeur statistique ajoutée à cette position.

2. Produit (10) selon la revendication 1,
**caractérisé en ce**
**que** le moyen de consigne (11) est configuré pour créer un masque numérique (50) de la posture prédéfinie et pour créer la consigne pour la personne (40) afin qu'elle prenne la posture prédéfinie à l'intérieur du masque numérique.

3. Produit (10) selon l'une quelconque des revendications précédentes,
**caractérisé en ce**
**que** le produit (10) est mis en œuvre sous la forme d'une application pour une exécution sur un dispositif intelligent.

4. Système de réglage (20) sur lequel est installé un produit (10) selon l'une quelconque des revendications précédentes pour le réglage électrique d'un siège de véhicule (31), présentant une unité de transmission de signal (23) pour transmettre le signal de réglage pour le réglage du siège de véhicule (31) au véhicule (30).

5. Système de réglage (20) selon la revendication 4,
**caractérisé en ce**
**qu'**un dispositif d'affichage (21) est prévu pour afficher la consigne créée par le moyen de consigne (11) pour prendre la posture prédéfinie et/ou le masque numérique (50).

6. Système de réglage (20) selon l'une quelconque des revendications 4 à 5,
**caractérisé en ce**
**qu'**une caméra (22) est prévue pour photographier la personne (40), dans lequel l'unité de consigne (11) est configurée pour créer une recommandation de position pour la caméra (22) pour photographier la personne (40) et pour l'afficher avec une indication de distance et/ou une indication d'angle de la caméra (22) par rapport à la personne et/ou par rapport au sol sur un dispositif d'affichage (21) du système de réglage (20).

7. Système de réglage (20) selon l'une quelconque des revendications 4 à 6,
**caractérisé en ce**
**que** le moyen de consigne (11) est configuré pour créer et afficher une confirmation d'une posture correctement prise et/ou pour créer et afficher une remarque concernant une posture non correctement prise sur le dispositif d'affichage (21).

8. Véhicule (30), qui est configuré pour le réglage électrique d'un siège de véhicule (31) du véhicule (30) avec un système de réglage (20) selon l'une quelconque des revendications 4 à 7, présentant une unité de réception de signal (32) pour recevoir le signal de réglage ainsi qu'une unité de réglage de siège (33), qui est en liaison de signal avec l'unité de réception de signal (32) pour le réglage électrique du siège de véhicule (31) sur la base du signal de réglage.

9. Véhicule (30) selon la revendication 8,
**caractérisé en ce**
**que** la caméra (22) est intégrée dans la zone extérieure du véhicule (30).
